# FASCICULE DE BREVET EUROPEEN

(11) **EP 3 145 386 B1**
(45) Date de publication et mention de la délivrance du brevet: **18.07.2018**
(21) Numéro de dépôt: 15726239.5
(22) Date de dépôt: 19.05.2015
(51) Int. Cl.: A61B 3/00, A61B 3/113, A61B 3/14, G02C 13/00

(54) **PROCÉDÉ DE DÉTERMINATION D'AU MOINS UN PARAMÈTRE DE COMPORTEMENT VISUEL D'UN INDIVIDU**
VERFAHREN ZUR BESTIMMUNG MINDESTENS EINES PARAMETERS DES VISUELLEN VERHALTENS EINES INDIVIDUUMS
METHOD OF DETERMINING AT LEAST ONE PARAMETER OF VISUAL BEHAVIOUR OF AN INDIVIDUAL

(30) Priorité: 20.05.2014 FR 1454547
(43) Date de publication de la demande: 29.03.2017
(73) Titulaire: Essilor International, 94220 Charenton-le-Pont (FR)
(72) Inventeur: BONNIN, Thierry, 94227 Charenton-le-Pont, Cedex (FR); ESCALIER, Guilhem, 94227 Charenton-le-Pont, Cedex (FR); HADDADI, Ahmed, 94227 Charenton-le-Pont, Cedex (FR)
(74) Mandataire: Jacobacci Coralis Harle
(86) Numéro de dépôt international: PCT/FR2015/051314
(87) Numéro de publication internationale: WO 2015/177459

(56) Documents cités:
- WO-A1-2006/106248
- WO-A1-2010/119190
- FR-A1- 2 914 173
- FR-A1- 2 932 675

## Description

### DOMAINE TECHNIQUE AUQUEL SE RAPPORTE L'INVENTION

La présente invention concerne le domaine de la personnalisation des lentilles ophtalmiques destinées à être montées dans une monture de lunettes.

Elle concerne plus précisément un procédé de détermination d'au moins un paramètre de comportement visuel d'un individu.

### ARRIERE-PLAN TECHNOLOGIQUE

On connaît des procédés permettant la personnalisation des lentilles ophtalmiques d'un équipement optique destiné à un individu en fonction de paramètres liés à l'individu et à la monture de lunettes choisie par celui-ci.

Un tel procédé est par exemple basé sur la détermination de la position du centre de rotation de chaque oeil de l'individu par rapport à la monture positionnée sur sa tête. Il est alors possible de déterminer précisément la position du centre optique de chaque lentille ophtalmique dans la monture choisie de manière à ce que la lentille ophtalmique soit correctement positionnée devant les yeux de l'individu.

Ici, la position du centre de rotation de l'oeil est déterminée par rapport à la lentille ophtalmique, de manière statique, dans des conditions éloignées des conditions naturelles du porté de la monture, sans prendre en compte la posture de l'individu.

Les procédés connus ne permettent pas par exemple de déterminer la position du centre de rotation de l'oeil de l'individu par rapport à la posture du corps de l'individu, dans des conditions proches de la vie quotidienne de l'individu et éventuellement pendant un mouvement de cet individu.

En effet, les dispositifs et procédés existant qui permettent de déterminer la posture du corps par rapport au dispositif de capture d'image ne disposent pas d'une précision suffisante pour permettre la détermination de la position des centres de rotation des yeux par rapport au corps. On connait du document WO 2010/119190 un procédé de détermination d'au moins un paramètre de comportement visuel d'un individu, comprenant une étape de détermination de la position du centre de rotation d'au moins un oeil de l'individu dans un référentiel lié à la tête de l'individu.

### OBJET DE L'INVENTION

Afin de remédier aux inconvénients précités de l'état de la technique, la présente invention propose un procédé de détermination d'au moins un paramètre de comportement visuel d'un individu permettant de prendre en compte la posture du corps de l'individu.

Plus particulièrement, on propose selon l'invention un procédé de détermination d'au moins un paramètre de comportement visuel d'un individu conforme à la revendication 1.

Il est ainsi possible de personnaliser les lentilles ophtalmiques destinées à la monture de lunettes choisie par cet individu en fonction de ce paramètre de comportement visuel.

On remarque que le paramètre de comportement visuel est déterminé dans le cadre du deuxième référentiel, par exemple dans un contexte où l'individu a un comportement plus naturel ; cette détermination peut toutefois ainsi utiliser la position du centre de rotation de l'oeil (dans le deuxième référentiel), déterminée initialement avec précision dans le premier référentiel, par exemple dans un environnement professionnel.

Le paramètre de comportement visuel est ainsi déterminé en tenant compte de la position du centre de rotation de l'oeil dans le deuxième référentiel où la posture de la tête et du corps de l'individu correspond à celle qu'il a naturellement, dans la situation de vie concernée.

Par ailleurs, la position et/ou l'orientation de la zone remarquable de ladite partie du corps de l'individu sont par exemple prédéterminées dans le premier référentiel conformément à la revendication 3, ce qui permet d'obtenir, sur la base de l'image capturée dans le deuxième référentiel, la position relative du premier référentiel et du deuxième référentiel.

D'autres caractéristiques non limitatives et avantageuses du procédé conforme à l'invention sont énoncées dans les revendications 2 et 4 à 18. D'autres caractéristiques non limitatives et avantageuses du procédé sont :
- à l'étape de capture, on capture au moins une séquence de dix images pendant que l'individu est libre de ses mouvements ;
- la situation de vie mentionnée ci-dessus est l'une des situations de vie suivantes :
   - situations de lecture et/ou écriture,
   - situations de repos,
   - situations de marche à pied,
   - situations dans laquelle l'individu monte ou descend un escalier ;
- à l'étape de capture, on place le dispositif de capture d'image de telle sorte que l'individu en mouvement reste sensiblement orienté de trois quarts par rapport à ce dispositif de capture d'image dans la situation de vie correspondante ;
- au moins l'étape de capture est réalisée par l'individu dans son environnement habituel.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique de profil de la tête de l'individu pendant une capture d'image à l'étape a) du procédé selon l'invention, et,
- la figure 2 est une vue schématique du corps de l'individu pendant une capture d'image à l'étape c) du procédé selon l'invention.

Le procédé selon l'invention comporte les étapes suivantes :
a) on détermine la position du centre de rotation d'au moins un oeil de l'individu dans un premier référentiel lié à la tête de l'individu,
b) on place l'individu dans une situation de vie dans laquelle la posture de la tête et du corps de l'individu est libre,
c) on capture, à l'aide d'un dispositif de capture d'image, au moins une image d'au moins une partie du corps de l'individu,
d) on détermine, en fonction de l'image capturée à l'étape c), la position et l'orientation de ladite partie du corps de l'individu dans un deuxième référentiel,
e) on détermine la position et l'orientation, dans le deuxième référentiel, du premier référentiel lié à la tête de l'individu, en recherchant la position, dans le deuxième référentiel, d'une zone remarquable de ladite partie du corps de l'individu dont la position et l'orientation dans le premier référentiel sont prédéterminées,
f) on en déduit la position du centre de rotation de l'oeil dans le deuxième référentiel,
g) on en déduit le paramètre de comportement visuel recherché.

En pratique, le procédé est mise en oeuvre par une unité de traitement informatique.

Cette unité de traitement informatique utilise la position du centre de rotation d'au moins un oeil de l'individu dans le premier référentiel lié à la tête de l'individu, présente en mémoire dans cette unité de traitement, transmise à cette unité de traitement ou bien déterminée par l'unité de traitement à partir de mesures effectuées sur l'individu, notamment à partir d'images en deux ou trois dimensions de la tête de cet individu réalisée à l'étape a) par un premier dispositif de capture d'images en deux ou trois dimensions.

Elle utilise également d'autres mesures effectuées sur l'individu comprenant au moins une image en deux ou trois dimensions d'une partie du corps de l'individu capturée à l'étape c), capturée avec un deuxième dispositif de capture d'image qui peut être le même que celui utilisé éventuellement à l'étape a).

Par partie du corps, on entend une partie du corps comprenant au moins une partie autre que la tête de l'individu. Plus précisément, il s'agit de préférence d'une partie du corps située en dessous de la ligne des épaules de l'individu.

Cette partie du corps peut comporter la tête de l'individu. Elle comporte en outre de préférence le cou et/ou une partie du buste de l'individu.

Elle peut comporter l'ensemble du corps et de la tête de l'individu, comme expliqué plus loin.

A partir des images en deux ou trois dimensions obtenues à l'étape c), l'unité de traitement informatique détermine la posture de ladite partie du corps dans le deuxième référentiel, c'est-à-dire la position et l'orientation de cette partie du corps dans le deuxième référentiel.

Ce deuxième référentiel peut être un référentiel lié au dispositif de capture d'image ou un référentiel absolu de l'espace, ou un référentiel lié à un élément de l'environnement de l'individu.

Ensuite, l'unité de traitement informatique est programmée pour repositionner le premier référentiel lié à la tête de l'individu par rapport au deuxième référentiel, de manière à permettre la détermination de la position des centres de rotation des yeux de l'individu par rapport à la partie du corps de l'individu.

L'unité de traitement informatique en déduit alors au moins un paramètre de comportement visuel de l'individu. Grâce aux étapes précédentes, cette détermination tient compte de la posture du corps et de la tête de l'individu dans sa situation de vie.

Les différentes étapes vont maintenant être décrites plus en détail.

### Etape a)

Lors de cette étape, la position du centre de rotation CRO d'au moins un oeil OD de l'individu est déterminée dans le premier référentiel lié à la tête TS de l'individu, par toute technique connue de l'homme du métier.

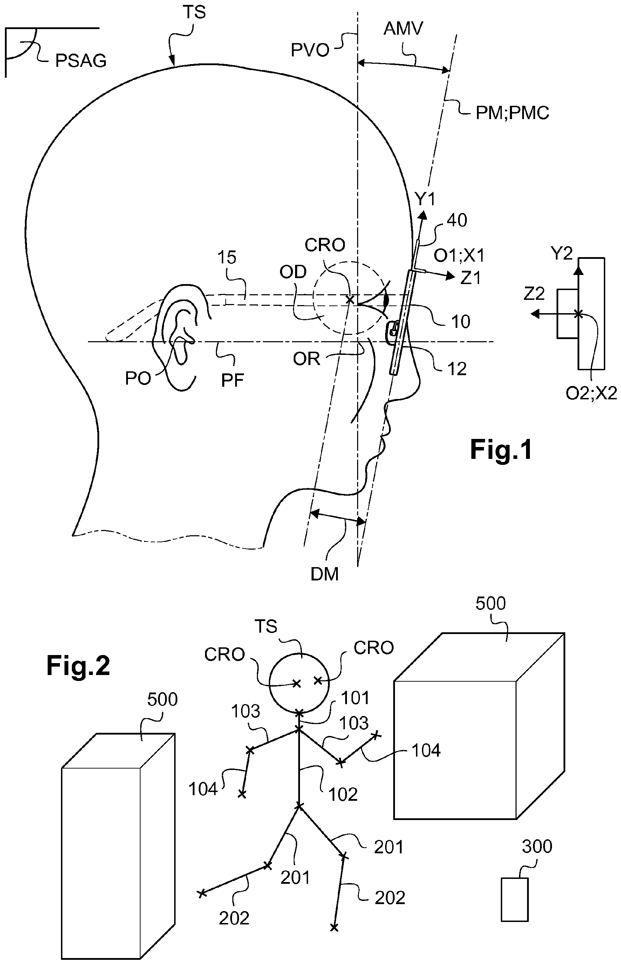

La tête de l'individu TS est par exemple représentée sur la figure 1.

Le plan de Francfort PF de la tête TS de l'individu est défini comme le plan passant par les points orbitaires inférieurs OR et le porion PO de l'individu, le porion étant le point du crâne le plus élevé du conduit auditif, qui correspond au tragion de l'oreille.

Le plan de Francfort PF est horizontal dans une position orthostatique, position dans laquelle il réalise un minimum d'efforts. L'axe de regard de l'individu est l'axe de regard primaire, c'est-à-dire qu'il regarde droit devant lui.

On définit un plan sagittal PSAG de la tête TS de l'individu comme étant le plan vertical passant par la médiatrice des deux yeux. La médiatrice des yeux est l'axe passant au milieu du segment défini par les centres de rotation des deux yeux, perpendiculaire à ce segment, et parallèle au plan de Francfort PF.

De préférence, la détermination de ce centre de rotation est réalisée à partir d'une mesure effectuée sur le visage de l'individu.

Cette mesure peut notamment comprendre la capture d'une ou plusieurs images en deux dimensions de la tête de l'individu ou l'acquisition d'une ou plusieurs représentations en trois dimensions de la tête de l'individu grâce à un premier dispositif de capture d'image.

Il s'agit ici de préférence de la capture d'une image ou de l'acquisition d'une représentation présentant une haute résolution, par exemple d'au moins 640 x 480 (VGA).

Ainsi, selon un premier mode de réalisation de l'étape a), on détermine la position du centre de rotation CRO de l'oeil à partir de deux images en deux dimensions de la tête de l'individu dans deux postures de tête différentes pour une même direction de regard par rapport au premier dispositif de capture d'image utilisé pour la capture de ces images.

Le principe de cette détermination est exposé en détails par exemple dans le document FR2914173, dont un équivalent en anglais est le document US20100128220.

En particulier, selon ce premier mode de réalisation de l'étape a),
- on réalise, à l'aide du premier dispositif de capture d'image, la capture d'au moins deux images de la tête de l'individu pour lesquelles les postures de la de la tête et du corps de l'individu est libre,
   c) on capture, à l'aide d'un dispositif de capture d'image, au moins une image d'au moins une partie du corps de l'individu,
   d) on détermine, en fonction de l'image capturée à l'étape c), la position et l'orientation de ladite partie du corps de l'individu dans un deuxième référentiel,
   e) on détermine la position et l'orientation, dans le deuxième référentiel, du premier référentiel lié à la tête de l'individu, en recherchant la position, dans le deuxième référentiel, d'une zone remarquable de ladite partie du corps de l'individu dont la position et l'orientation dans le premier référentiel sont prédéterminées,
   f) on en déduit la position du centre de rotation de l'oeil dans le deuxième référentiel,
   g) on en déduit le paramètre de comportement visuel recherché.

En pratique, le procédé est mise en oeuvre par une unité de traitement informatique.

Cette unité de traitement informatique utilise la position du centre de rotation d'au moins un oeil de l'individu dans le premier référentiel lié à la tête de l'individu, présente en mémoire dans cette unité de traitement, transmise à cette unité de traitement ou bien déterminée par l'unité de traitement à partir de mesures effectuées sur l'individu, notamment à partir d'images en deux ou trois dimensions de la tête de cet individu réalisée à l'étape a) par un premier dispositif de capture d'images en deux ou trois dimensions.

Elle utilise également d'autres mesures effectuées sur l'individu comprenant au moins une image en deux ou trois dimensions d'une partie du corps de l'individu capturée à l'étape c), capturée avec un deuxième dispositif de capture d'image qui peut être le même que celui utilisé éventuellement à l'étape a).

Par partie du corps, on entend une partie du corps comprenant au moins une partie autre que la tête de l'individu. Plus précisément, il s'agit de préférence d'une partie du corps située en dessous de la ligne des épaules de l'individu.

Cette partie du corps peut comporter la tête de l'individu. Elle comporte en outre de préférence le cou et/ou une partie du buste de l'individu.

Elle peut comporter l'ensemble du corps et de la tête de l'individu, comme expliqué plus loin.

A partir des images en deux ou trois dimensions obtenues à l'étape c), l'unité de traitement informatique détermine la posture de ladite partie du corps dans le deuxième référentiel, c'est-à-dire la position et l'orientation de cette partie du corps dans le deuxième référentiel.

Ce deuxième référentiel peut être un référentiel lié au dispositif de capture d'image ou un référentiel absolu de l'espace, ou un référentiel lié à un élément de l'environnement de l'individu.

Ensuite, l'unité de traitement informatique est programmée pour repositionner le premier référentiel lié à la tête de l'individu par rapport au deuxième référentiel, de manière à permettre la détermination de la position des centres de rotation des yeux de l'individu par rapport à la partie du corps de l'individu.

L'unité de traitement informatique en déduit alors au moins un paramètre de comportement visuel de l'individu. Grâce aux étapes précédentes, cette détermination tient compte de la posture du corps et de la tête de l'individu dans sa situation de vie.

Les différentes étapes vont maintenant être décrites plus en détail.

### Etape a)

Lors de cette étape, la position du centre de rotation CRO d'au moins un oeil OD de l'individu est déterminée dans le premier référentiel lié à la tête TS de l'individu, par toute technique connue de l'homme du métier.

La tête de l'individu TS est par exemple représentée sur la figure 1.

Le plan de Francfort PF de la tête TS de l'individu est défini comme le plan passant par les points orbitaires inférieurs OR et le porion PO de l'individu, le porion étant le point du crâne le plus élevé du conduit auditif, qui correspond au tragion de l'oreille.

Le plan de Francfort PF est horizontal dans une position orthostatique, position dans laquelle il réalise un minimum d'efforts. L'axe de regard de l'individu est l'axe de regard primaire, c'est-à-dire qu'il regarde droit devant lui.

On définit un plan sagittal PSAG de la tête TS de l'individu comme étant le plan vertical passant par la médiatrice des deux yeux. La médiatrice des yeux est l'axe passant au milieu du segment défini par les centres de rotation des deux yeux, perpendiculaire à ce segment, et parallèle au plan de Francfort PF.

De préférence, la détermination de ce centre de rotation est réalisée à partir d'une mesure effectuée sur le visage de l'individu.

Cette mesure peut notamment comprendre la capture d'une ou plusieurs images en deux dimensions de la tête de l'individu ou l'acquisition d'une ou plusieurs représentations en trois dimensions de la tête de l'individu grâce à un premier dispositif de capture d'image.

Il s'agit ici de préférence de la capture d'une image ou de l'acquisition d'une représentation présentant une haute résolution, par exemple d'au moins 640 x 480 (VGA).

Ainsi, selon un premier mode de réalisation de l'étape a), on détermine la position du centre de rotation CRO de l'oeil à partir de deux images en deux dimensions de la tête de l'individu dans deux postures de tête différentes pour une même direction de regard par rapport au premier dispositif de capture d'image utilisé pour la capture de ces images.

Le principe de cette détermination est exposé en détails par exemple dans le document FR2914173, dont un équivalent en anglais est le document US20100128220.

En particulier, selon ce premier mode de réalisation de l'étape a),
- on réalise, à l'aide du premier dispositif de capture d'image, la capture d'au moins deux images de la tête de l'individu pour lesquelles les postures de la tête de l'individu par rapport au premier dispositif de capture d'image sont différentes et pour lesquelles l'individu fixe du regard un point de visée de position prédéterminée et fixe,
- on détermine les directions de regard de l'individu correspondant à chacune des deux images,
- on en déduit la position du centre de rotation de l'oeil de l'individu dans le premier référentiel lié à la tête de l'individu.

Les images de la tête de l'individu capturées sont transmises à l'unité de traitement informatique.

Les images capturées peuvent être traitées en temps réel ou après la capture de toutes les images.

A partir de ces images capturées à l'étape a), l'unité de traitement informatique détermine la position du centre de rotation CRO d'au moins un oeil de l'individu dans le premier référentiel lié à la tête de l'individu.

A titre d'exemple, il est possible d'identifier les images d'un point remarquable de l'oeil de l'individu, par exemple de la pupille de l'oeil de l'individu sur deux images capturées alors que l'individu fixe des yeux une cible dont la position par rapport au premier dispositif de capture d'image est différente pour chaque image capturée.

La position de la cible fixée du regard pendant les deux captures d'image étant connue par rapport au premier dispositif de capture d'image, on en déduit la position du centre de rotation de l'oeil CRO comme étant l'intersection des droites passant par la cible et la pupille de l'oeil pour chaque image capturée.

On peut également déterminer pour chaque direction de regard la posture de la tête de l'individu correspondante, c'est-à-dire sa position et son orientation dans un référentiel lié au premier dispositif de capture d'image. Un couple de données associant la posture de la tête et la direction de regard est stocké en correspondance avec l'image.

En pratique, le premier dispositif de capture d'images est alors de préférence un dispositif du type appareil photographique ou camera. Il est par exemple solidaire d'un écran d'affichage sur lequel lesdites cibles constituant les points de visée sont affichées. Il peut s'agir par exemple d'une tablette comportant une caméra qui constitue le premier dispositif de capture d'image. Les cibles peuvent comprendre soit une pluralité d'images statiques apparaissant à différents endroits de l'écran, soit une image dynamique se déplaçant sur l'écran.

L'individu peut être éventuellement équipé de montures de lunettes 10 et/ou d'un système de repérage 40 destiné à permettre la détermination de la position de la tête de l'individu dans l'espace à partir d'une image capturée de la tête de l'individu équipée du système de repérage. Ce système de repérage est décrit en détails dans le document FR2914173, page 7, ligne 5 à page 10, ligne 8. Il ne sera donc pas décrit plus en détail ici.

La monture 10 peut être cerclée ou percée. Dans l'exemple représenté sur la figure 1, elle est cerclée, et comporte donc deux cercles 12 reliés par un pont et destinés à accueillir les lentilles ophtalmiques, et deux branches 15 qui sont destinées à reposer sur les oreilles de l'individu.

Le système de repérage 40 présente des caractéristiques géométriques prédéterminées, qui permettent, à partir d'une image capturée de la tête de l'individu sur laquelle apparaît ce système de repérage, de déterminer la position et l'orientation de la tête de l'individu dans l'espace, dans ledit référentiel lié au dispositif de capture d'image. Ce système de repérage permet donc de déterminer la position et l'orientation du premier référentiel lié à la tête de l'individu dans le référentiel lié au dispositif de capture d'image.

Le premier référentiel (O1, X1, Y1, Z1) lié à la tête de l'individu peut d'ailleurs être lié au système de repérage 40, comme représenté sur la figure 1 de manière schématique. Sur cet exemple, le centre du référentiel est disposé au milieu du système de repérage, qui est situé dans le plan sagittal de la tête de l'individu. L'axe (O1,Y1) s'étend dans le plan moyen de la monture PM, qui est ici confondu avec le plan moyen de chaque cercle PMC.

L'axe (O1, X1) est parallèle au segment reliant les centres de rotation des yeux de l'individu et l'axe (O1, Z1) est perpendiculaire aux deux axes (O1, X1) et (O1, Y1).

Les caractéristiques géométriques du système de repérage 40 donnent accès à un facteur d'échelle de chaque image capturée et aux angles de rotation de la tête de l'individu par rapport au dispositif de capture d'image.

Un exemple de référentiel (O1, X2, Y2, Z2) lié au dispositif de capture d'image est représenté sur la figure 1 dans le cas où il s'agit d'une caméra ou d'un appareil photographique. Le centre 02 de ce référentiel est disposé par exemple au centre du capteur de ce dispositif. L'axe (02, Z2) s'étend le long de l'axe optique. Les axes(O2, Y2) et (O2, X2) s'étendent dans le plan perpendiculaire à l'axe optique.

Selon un deuxième mode de réalisation de l'étape a), on détermine la position du centre de rotation de l'oeil CRO en fonction d'une position moyenne prédéterminée de ce centre de rotation.

Il peut s'agir par exemple de sa position moyenne par rapport à la face arrière d'une lentille ophtalmique équipant une monture de lunettes placée sur la tête de l'individu. A cet effet, on considère par exemple que le centre de rotation est situé à une distance moyenne DM égale à 27 millimètres de la face arrière de la lentille ophtalmique. Il est ainsi possible de déterminer la position du centre de rotation à partir d'une capture d'image de la tête de l'individu en deux dimensions de profil (voir par exemple la figure 1).

Selon un troisième mode de réalisation de l'étape a), on détermine les coordonnées en trois dimensions des centres de rotation des yeux à partir d'images en trois dimensions de la tête de l'individu.

Dans le cas d'images en trois dimensions, on parlera dans la suite également de représentations en trois dimensions.

En pratique, la mise en oeuvre de ce deuxième mode de réalisation est proche de celle du premier mode de réalisation.

On réalise par exemple l'acquisition d'au moins deux représentations en trois dimensions de la tête de l'individu pendant que celui-ci fixe du regard une cible dont la position est connue par rapport audit premier dispositif de capture d'image et pour des angles de la tête différents par rapport au premier dispositif de capture d'image.

Ce premier dispositif de capture d'image est ici un dispositif d'acquisition de la représentation en trois dimensions.

La cible est par exemple située droit devant l'individu.

Pour chaque représentation en trois dimensions, on détermine la position de la pupille de l'oeil de l'individu et on détermine la direction du regard comme la droite reliant la pupille à la cible.

En superposant les deux représentations, on détermine le centre de rotation de l'oeil comme l'intersection entre les deux directions de regard déterminées.

Les centres de rotation des yeux de l'individu peuvent ainsi être repérés par leurs coordonnées dans un référentiel lié au premier dispositif d'acquisition de la représentation en trois dimensions.

Ces coordonnées peuvent être ensuite transposées dans le premier référentiel lié à la tête de l'individu.

Le premier référentiel peut être lié à une monture de lunette préalablement posée sur la tête de l'individu, à des points particuliers du visage de cet individu, ou encore à un système de repérage du type de celui décrit dans le document FR2914173.

Les représentations en trois dimensions peuvent être obtenues par une technique de capture d'images stéréoscopiques, ou par une technique d'acquisition en trois dimensions du type scanner en trois dimensions, basée par exemple sur une lumière structurée.

Ces derniers dispositifs d'acquisition d'une représentation en trois dimensions comprennent des moyens de projection de lumière structurée, comportant par exemple un motif tel qu'une figure de moiré, sur la tête de l'individu pendant que des moyens de capture d'image enregistrent une ou plusieurs images en deux dimensions de la tête de l'individu. Le motif étant connu, le traitement de ces images permet de déterminer la représentation en trois dimensions.

Ces représentations en trois dimensions peuvent également être obtenues par une méthode d'imagerie plénoptique. Il s'agit d'une méthode d'imagerie multi-focale permettant de récupérer plusieurs points de vue sur un seul capteur. Ce type d'image dit "light-field" permet de déterminer une représentation en trois dimensions de la tête de l'individu.

Selon un quatrième mode de réalisation de l'étape a), la position du centre de rotation de l'oeil de l'individu est déterminée à partir d'une base de données morphologiques de l'individu.

Dans ce cas, à l'étape a), un ensemble de données relatives à l'individu, provenant par exemple du traitement d'images ou de représentations en trois dimensions enregistrées au préalable, est récupéré et exploité par l'unité de traitement informatique pour déterminer la position des centres de rotation.

De manière générale, on peut envisager que l'étape a) soit réalisée par une personne spécialisée dans l'optométrie, telle l'opticien, à l'aide d'un dispositif dédié, ou sous la responsabilité de l'individu lui-même, sans intervention d'une personne spécialisée dans l'optométrie et/ou à l'aide d'un dispositif d'usage courant.

Elle est par exemple réalisée chez l'opticien, avec les moyens dédiés possédés par lui, et dans les conditions particulières associées au lieu de travail de l'opticien, notamment en termes de positionnement de l'individu, de l'éclairage, des dispositifs de mesure et du temps court dédié à la mesure.

### Etape b)

On place l'individu dans une situation de vie dans laquelle la posture de la tête et du corps de l'individu est libre.

Il s'agit en particulier de l'une des situations de vie suivantes :
- situations de lecture et/ou écriture,
- situations de repos,
- situations de marche à pied,
- situations dans laquelle l'individu monte ou descend un escalier.

Les situations de lecture/écriture sur une chaise, un tabouret, un canapé, devant un bureau sont destinées à permettre la détermination des distances de lecture, d'un port de tête (défini par un angle d'abaissement et un angle de roulis) ou d'un angle d'inclinaison de la tête pendant une tâche de lecture.

Les situations de repos permettent de déterminer une posture globale du corps pouvant influencer des paramètres de vision. Il est en particulier important de déterminer si l'individu présente une posture voutée ou droite au repos.

Plusieurs situations dynamiques comme la marche, la montée et/ou la descente des escaliers permettent par exemple d'observer des comportements d'oscillation ou d'hésitation de l'individu.

A l'étape b), l'individu évolue de préférence dans son environnement habituel, c'est-à-dire par exemple chez lui, à son bureau, dans sa voiture ou à pied dans son quartier.

Cependant, on peut également envisager qu'à l'étape b), l'individu évolue dans le magasin de l'opticien. Différentes situations de vie peuvent être alors aménagées au sein du magasin de l'opticien, de manière à proposer à l'individu de s'asseoir pour lire ou regarder un écran dans un canapé ou de monter ou descendre un escalier.

### Etape c)

On capture, à l'aide d'un deuxième dispositif de capture d'image, au moins une image d'au moins une partie du corps de l'individu. Il peut s'agir d'images en deux ou trois dimensions. Dans le cas d'images en trois dimensions, on parlera également de représentations en trois dimensions.

Lorsqu'un premier dispositif de capture d'images en deux ou trois dimensions est utilisé à l'étape a), le deuxième dispositif de capture d'image utilisé lors de l'étape c) peut être celui de l'étape a) ou distinct de celui de l'étape a). Le dispositif peut éventuellement être en mode d'acquisition différent (multirésolution) dans les deux cas.

De préférence, l'image en deux ou trois dimensions capturée à l'étape c) comprend au moins les images en deux dimensions ou les représentations en trois dimensions de la tête de l'individu et d'une partie de son buste.

De préférence, cette image comprend le corps entier avec la tête de l'individu.

Selon un premier mode de réalisation de l'étape c), ladite image est une représentation en trois dimensions de la partie du corps de l'individu.

Les représentations en trois dimensions peuvent être obtenues, comme à l'étape a), par une technique de capture d'images stéréoscopiques, ou par une technique d'acquisition en trois dimensions du type scanner en trois dimensions, basée par exemple sur une lumière structurée.

Le deuxième dispositif de capture d'image est alors un dispositif d'acquisition d'une représentation en trois dimensions comprenant des moyens de projection de lumière structurée, c'est-à-dire une lumière comportant un motif tel qu'une figure de moiré, sur la tête de l'individu pendant que des moyens de capture d'image enregistre une ou plusieurs images en deux dimensions de la tête de l'individu. Le motif étant connu, le traitement de ces images permet de déterminer la représentation en trois dimensions.

Un dispositif du type « Kinect », dont le principe de fonctionnement est décrit dans le document US20100118123, peut par exemple être utilisé.

Ces représentations en trois dimensions peuvent également être obtenues par une méthode d'imagerie plénoptique. Il s'agit d'une méthode d'imagerie multi-focale permettant de récupérer plusieurs points de vue sur un seul capteur. Ce type d'image dit "light-field" permet de déterminer une représentation en trois dimensions du corps de l'individu.

Selon un deuxième mode de réalisation de l'étape c), ladite image capturée à l'étape c) est une image en deux dimensions capturée à l'aide d'un deuxième dispositif de capture d'image tel qu'un appareil de photographie ou une caméra.

Quel que soit le mode de réalisation envisagé, l'orientation de l'individu par rapport au deuxième dispositif de capture d'images permet d'observer le regard de l'individu et l'environnement de l'individu. Par exemple dans le cas où la tâche de l'individu consiste à monter ou descendre des escaliers, il faut pouvoir observer la posture de la tête et du corps de l'individu et les marches de l'escalier.

La posture idéale correspond le plus souvent à une orientation du corps et de la tête de l'individu dans laquelle celui-ci est vu de trois quarts par le deuxième dispositif de capture d'image.

On place alors de préférence le deuxième dispositif de capture d'image de telle sorte que l'individu en mouvement reste sensiblement orienté de trois quarts par rapport à ce deuxième dispositif de capture d'image dans la situation de vie correspondante.

En pratique, la posture de trois quarts implique que le plan sagittal de la tête de l'individu est orienté selon un angle compris entre 20 et 50 degrés par rapport à un plan perpendiculaire au plan de capture d'image du deuxième dispositif de capture d'image, dans le cas d'un deuxième dispositif de capture d'image en deux dimensions.

En outre, on capture de préférence à l'étape c) une séquence d'images en deux ou trois dimensions pendant une durée prédéterminée.

On capture plus précisément au moins une séquence de dix images pendant que l'individu est libre de ses mouvements.

On peut envisager que l'étape c) soit réalisée par une personne spécialisée dans l'optométrie, telle l'opticien, à l'aide d'un dispositif dédié, ou sous la responsabilité de l'individu lui-même, sans intervention d'une personne spécialisée dans l'optométrie et/ou à l'aide d'un dispositif d'usage courant.

Elle est par exemple réalisée chez l'opticien, avec les moyens dédiés possédés par lui, et dans les conditions particulières associées au lieu de travail de l'opticien, notamment en termes de positionnement de l'individu, de l'éclairage, des dispositifs de mesure et du temps court dédié à la mesure.

En pratique, afin de réaliser les captures d'images en deux ou trois dimensions de l'étape c), l'individu suit alors un protocole de mesure annoncé par l'opticien.

En variante, l'étape c) est réalisée sous la responsabilité de l'individu lui-même, dans l'environnement habituel de l'individu, à l'aide de dispositifs de mesure d'usage courant, comme un appareil photographique, une caméra ou une webcam, avec la possibilité d'y consacrer un temps beaucoup plus long que celui dédié aux mesures généralement réalisées chez l'opticien.

En pratique, afin de réaliser les captures d'images en deux ou trois dimensions de l'étape c), l'individu suit alors un protocole de mesure qui peut être annoncé par une fiche expliquant le protocole à suivre ou un site internet présentant l'explication du déroulement de la mesure. A chaque étape de ce protocole de mesure, une interaction avec le deuxième dispositif de capture d'image utilisé peut être demandée à l'individu.

### Etape d)

On détermine, en fonction de l'image capturée à l'étape c), la position et l'orientation de ladite partie du corps de l'individu dans le deuxième référentiel.

Ce deuxième référentiel peut être lié audit deuxième dispositif de capture d'image ou un référentiel absolu, ou lié à l'environnement de l'individu, qui est non lié au premier dispositif de capture d'image.

Cette étape est réalisée par l'unité de traitement informatique à partir des images en deux ou trois dimensions capturées à l'étape c).

Il s'agit de déterminer un modèle en trois dimensions de ladite partie du corps de l'individu.

Dans le cas où la représentation en trois dimensions du corps de l'individu à l'étape c) est acquise grâce à un dispositif du type « Kinect » 300 (figure 2), il est en effet possible, de manière connue, de traiter les représentations en trois dimensions acquises de manière à relever les positions et les angles de certains segments 101, 102, 103, 104, 201, 202 du corps préalablement définis par l'utilisateur.

Dans le cas d'un module Kinect par exemple, les segments repérés du corps sont définis et modélisés au niveau du module OpenNI.

Globalement, le deuxième dispositif d'acquisition donne la position et l'orientation des parties du corps dans l'espace. Il permet de suivre en temps réel le mouvement du corps de l'individu dans un volume de l'espace assez important.

La résolution est de l'ordre de 0,5 centimètres. Cette résolution permet notamment d'identifier le système de repérage disposé sur la tête de l'individu par exemple.

Comme cela est représenté schématiquement sur la figure 2, on définit par exemple les segments du corps suivants : le cou 101, le buste 102, les bras 103, les avant-bras 104, les cuisses 201, les jambes 202 et éventuellement les mains et les pieds.

Chaque segment comprend au moins une extrémité associée à une articulation du corps et possédant au moins un degré de liberté par rapport au segment voisin.

Par exemple, on peut notamment prendre comme référentiel les 4 points suivants : acromion gauche (LAC), acromion droite (RAC), manubrium (SJN), apophyse xyphoïde (SXS). Dans le cas de l'utilisation d'un module Kinect, on utilise par exemple les points suivants : SHOULDER_RIGHT, SHOULDER_LEFT, SPINE_SHOULDER, SPINE_MID.

La position des articulations de l'individu et les angles des segments déterminés permettent d'établir un modèle en trois dimensions de ladite partie du corps de l'individu.

Sur la figure 1, l'environnement de l'individu est schématiquement représenté par les éléments de référence 500.

### Etape e)

On détermine la position et l'orientation, dans le deuxième référentiel, du premier référentiel lié à la tête de l'individu, en recherchant la position, dans le deuxième référentiel, d'une zone remarquable de ladite partie du corps de l'individu dont la position et l'orientation dans le premier référentiel sont prédéterminées.

Cette zone remarquable est constituée par exemple par une pluralité de points remarquables liés à ladite partie du corps de l'individu.

Cette zone remarquable peut être constituée par le cou de l'individu.

Le cou peut en effet être visible sur l'image de la tête de l'individu capturée à l'étape a) et sur l'image de la partie du corps de l'individu capturée à l'étape c).

Les points remarquables de la zone remarquable peuvent également être liés au système de repérage mentionné précédemment et monté sur la tête de l'individu mentionné précédemment, à une monture de lunette disposée sur sa tête ou par à un ou plusieurs points remarquables du visage de l'individu.

Ainsi, de préférence, à l'étape a), la tête de l'individu est munie d'une paire de lunettes surmontée d'un système de repérage, à l'étape c), la partie du corps capturée comprend la tête de l'individu munie de ce système de repérage, et, à l'étape e), la zone remarquable est constituée par ce système de repérage.

La méthode appelée « méthode Poslt », publiée par Daniel F. DeMenthon et Larry S. Davis en mai 1995 peut être utilisée dans ce but. Cette méthode permet de trouver la position et/ou l'orientation d'un objet à partir d'une seule image en deux dimensions et d'un modèle de l'objet en trois dimensions.

La mise en oeuvre de cette méthode nécessite de trouver au moins 4 points de correspondance entre l'objet en deux dimensions et le modèle en trois dimensions. Ces points de correspondances sont par exemple des points remarquable du système de repérage placé sur la tête de l'individu lors des étapes a) et c). Le modèle en trois dimensions est celui du corps de l'individu et l'objet en deux dimensions correspond aux positions du centre de rotation de l'oeil dans le premier référentiel. On se réfèrera par exemple au document US2012/321 134.

Il s'agit ici en fait de replacer les centres de rotation des yeux de l'individu dans le deuxième référentiel en trois dimensions de l'étape c), de manière à déterminer la position de ces centres de rotation par rapport au corps de l'individu.

### Etape f)

Une fois le premier référentiel positionné par rapport au deuxième référentiel, on détermine la position du centre de rotation de l'oeil dans ledit deuxième référentiel.

Il s'agit ici d'une étape de changement de référentiel.

### Etape g)

Le paramètre de comportement visuel de l'individu déterminé à l'étape g) est par exemple l'un des suivants :
- une zone d'usure correspondant à la situation de vie de l'étape b) pour une lentille correctrice à disposer dans une monture de lunettes destinée à équiper la tête de l'individu,
- un paramètre comportemental de l'individu précisant s'il bouge plus les yeux ou la tête lors d'une tâche visuelle déterminée,
- une distance de lecture moyenne,
- une posture naturelle de l'individu au repos,
- un comportement dynamique des yeux pendant la situation de vie choisie,
- une position d'une zone vision de près ou longueur de progression ou inset d'une lentille correctrice à disposer dans une monture de lunettes destinée à équiper la tête de l'individu,
- l'angle pantoscopique AMV d'une lentille correctrice à disposer dans une monture de lunettes destinée à équiper la tête de l'individu déterminé de manière à réduire les aberration d'astigmatisme.

L'angle pantoscopique AMV est défini comme l'angle entre le plan moyen de chaque cercle PMC de la monture de lunettes et le plan vertical oeil PVO, qui est le plan perpendiculaire au plan de Francfort passant par les centres de rotation CRO des yeux, mesuré en projection dans le plan sagittal de la tête de l'individu.

Par exemple, lors de l'étape g), on détermine la direction du regard de l'individu dans ladite situation de vie et on en déduit une zone d'usure de la lentille ophtalmique correspondant à cette situation de vie.

La zone d'usure de la lentille ophtalmique est définie comme étant une zone de l'espace représentative d'une distribution statistique d'un ensemble de points sur la lentille par lesquels passe le regard de l'individu lors d'une tâche particulière de vision ou pour une utilisation à une distance de travail prédéterminée. La zone d'usure peut être définie de manière équivalente soit spatialement, par une distribution statistique de points sur la lentille ophtalmique ou sur un autre plan de projection lié à la lentille ophtalmique ou au cercle de la monture correspondant, soit vectoriellement, par une distribution statistique de directions du regard. De manière alternative et simplifiée, la zone d'usure ZU peut aussi être définie de manière tabulée par une distribution statistique d'angles d'abaissement du regard dans le plan sagittal de l'individu.

L'angle d'abaissement du regard est défini comme l'angle entre la direction de regard et une direction de regard primaire prédéterminée en projection dans le plan sagittal de la tête de l'individu.

Cette direction de regard primaire prédéterminée correspond à la direction de regard de l'individu dans des conditions de vision de loin, c'est-à-dire dans des conditions où l'individu fixe un point distant d'au moins 5 mètres de lui.

Pour déterminer la direction du regard de l'individu, on identifie, sur l'image en deux ou trois dimensions capturée à l'étape c), l'image de la pupille de l'individu et on en déduit la direction du regard recherchée. La direction de regard est donc déterminée en fonction de la position du centre de rotation de l'oeil dans le premier référentiel déterminée à l'étape a).

Plus précisément, cette direction de regard est déterminée comme la droite reliant le centre de rotation de l'oeil et la pupille de cet oeil.

En variante, on pourrait utiliser un oculomètre.

Grâce à l'étape d), il est possible de définir cette direction de regard dans le premier ou le deuxième référentiel.

On peut également envisager que, pour déterminer la direction du regard de l'individu, on détermine la position dans le deuxième référentiel d'éléments cibles du regard appartenant à l'environnement de l'individu. Les éléments cibles du regard en question sont par exemple affichés sur un écran d'affichage et ont une position connue par rapport au deuxième dispositif de capture d'image.

Ces éléments cibles peuvent ainsi être constitués par un écran d'affichage, des marches d'escalier, les pages d'un livre, ou tout élément de l'environnement de l'individu.

La direction du regard peut alors être déterminée comme la droite reliant le centre de rotation de l'oeil et l'élément cible fixé du regard par l'individu.

On détermine ensuite par exemple l'intersection de la direction du regard et d'un plan moyen de la lentille ophtalmique destinée à être placée devant l'oeil de l'individu.

Le plan moyen de la lentille ophtalmique peut éventuellement être assimilé au plan moyen du cercle de la monture correspondant.

La position et l'orientation du plan moyen de la lentille sont par exemple prédéterminées lors d'une étape de calibration.

Cette détermination peut prendre en compte la forme de la monture de lunettes choisie par l'individu. En remplacement du plan moyen de la lentille, on peut également utiliser la face avant ou arrière de la lentille, ou une surface moyenne équidistante de cette face avant et arrière.

Dans le cas où la lentille ophtalmique considérée est une lentille progressive, la zone d'usure déterminée peut en particulier constituer la zone de vision de près ou de loin de la lentille ophtalmique progressive.

La puissance de la lentille ophtalmique progressive varie, de préférence continument, entre un point de référence pour la vision de loin situé dans la zone d'usure de la lentille en vision de loin et un point de référence pour la vision de près situé dans la zone d'usure pour la vision de près, le long d'une courbe appelée « courbe méridienne principale de progression » qui passe par ces deux points. Cette courbe méridienne principale de progression traverse ces deux zones d'usure ainsi qu'une zone d'usure intermédiaire située entre la zone d'usure en vision de près et la zone d'usure en vision de loin, suivant une direction globalement verticale.

On peut également déduire de manière avantageuse la longueur de progression et/ou l'inset de la lentille ophtalmique progressive en fonction de cette zone d'usure en vision de près et/ou de loin.

On définit la longueur de progression de la lentille ophtalmique comme la distance verticale entre la croix de montage et la position du point de référence en vision de près défini par le fabricant du verre.

La croix de montage est un point de référence pour le positionnement de la lentille devant l'oeil d'un individu dont la position est prédéfinie par le fabriquant de la lentille.

D'autres définitions peuvent être adoptées pour la longueur de progression. Elle peut être exprimée par rapport au point de référence du prisme ou au point de référence de vision de loin plutôt que par rapport à la croix de montage. Comme les positions respectives de ces points sont par ailleurs aussi données par le fabricant cette définition est équivalente à la précédente.

On définit l'inset de la lentille ophtalmique progressive comme le décalage horizontal entre le point de référence pour la vision de loin et le point de référence pour la vision de près. L'inset E est aussi appelé « déport interne ».

Le paramètre comportemental de l'individu précisant s'il bouge plus les yeux ou la tête lors d'une tâche visuelle déterminée peut être par exemple un coefficient oeil-tête défini par le rapport entre l'amplitude du déplacement d'un oeil de l'individu selon une direction déterminée dans une situation visuelle déterminée et l'amplitude maximale théorique du déplacement de cet oeil dans cette situation visuelle.

Ce paramètre comportemental peut également comprendre une amplitude du déplacement d'au moins un oeil de l'individu et/ou une amplitude du déplacement de la tête de l'individu dans cette situation visuelle déterminée.

La situation visuelle déterminée peut en particulier correspondre à une tâche de lecture.

Le coefficient oeil-tête correspond alors par exemple au rapport entre l'amplitude angulaire du déplacement de l'oeil de l'individu pendant la lecture d'un texte prédéterminé et l'amplitude maximale théorique de ce déplacement en fonction de la largeur du texte affiché et de la distance de lecture de l'individu.

On peut comparer de la même manière l'amplitude angulaire du déplacement de la tête de l'individu pendant la lecture et l'amplitude maximale théorique de ce déplacement.

Par ailleurs, la distance de lecture moyenne peut être obtenue par le traitement des images obtenues à l'étape c), en identifiant sur ces images l'image du support de lecture qui appartient à l'environnement de l'individu. Elle est par exemple définie comme la distance entre les centres de rotation des yeux et ce support de lecture.

La posture naturelle de l'individu au repos correspond à la position et l'orientation de la tête et d'au moins ladite partie du corps de l'individu lorsque celui-ci n'effectue pas une tâche visuelle particulière.

Le comportement dynamique des yeux pendant la situation de vie choisie est déterminé à l'aide d'un traitement statistique des images obtenues à l'étape c).

On capture à cet effet, à l'étape c), une séquence d'images en deux ou trois dimensions pendant une durée prédéterminée. On capture plus précisément au moins une séquence de dix images pendant que l'individu est libre de ses mouvements.

Il est ainsi possible d'en déduire l'évolution du paramètre de comportement visuel recherché pendant cette durée prédéterminée.

L'angle pantoscopique d'une lentille ophtalmique correctrice à disposer dans une monture de lunettes destinée à équiper la tête de l'individu est déterminé de manière à réduire les aberration d'astigmatisme.

De manière générale, quels que soient les détails de la mise en oeuvre envisagée, comme évoqué précédemment, les étapes a) et c) peuvent être réalisées en un même lieu, à l'aide d'un même dispositif de capture d'image ou en deux lieux différents, à l'aide de deux dispositifs de capture d'image différents.

De préférence, au moins les étapes b) et c) sont réalisées par l'individu dans son environnement habituel.

On envisage par exemple que l'environnement habituel comprend un environnement de travail, par exemple un bureau, un environnement de la maison, par exemple un canapé disposé devant la télé ou un fauteuil pour lire, un environnement de la voiture, par exemple un tableau de bord muni d'un volant.

L'environnement habituel de l'individu comprend aussi une partie du quartier où habite l'individu.

Dans le cas où on envisage la réalisation des étapes a) et c) en un même lieu, par exemple le magasin de l'opticien, on peut par exemple envisager que le premier et le deuxième dispositif de capture d'image soient intégrés à une colonne de mesure située dans le magasin de l'opticien.

La colonne intègre alors par exemple un dispositif de capture d'image en deux dimensions comme une caméra et un dispositif de capture d'image en trois dimensions, type Kinect, scan 3D. Les deux dispositifs peuvent également être combinés en un unique système d'acquisition utilisant par exemple la technologie Ray-light.

Le dispositif de capture d'image en trois dimensions peut éventuellement être piloté à distance par une télécommande par l'opticien afin d'effectuer les captures d'image adéquates dans l'environnement du magasin, par exemple pendant la marche ou la lecture de l'individu aux tables de vente.

Il est également possible d'envisager un placement étudié d'une pluralité de dispositif de capture d'image afin de couvrir l'ensemble de l'environnement du magasin. A titre d'exemple, le dispositif du type Kinect présente une portée de 8 mètres sur un angle solide d'environ 60 degrés. Il est donc possible de prévoir les lieux observés par ces dispositifs.

On peut également envisager que le premier dispositif de capture d'image soit intégré à une colonne de mesure, tandis que le deuxième dispositif de capture d'image comprend un dispositif portable que l'opticien ou l'individu transporte dans le magasin.

Dans le cas où l'on envisage la réalisation des étapes a) et c) en deux lieux différents, par exemple d'un côté dans le magasin de l'opticien et de l'autre chez l'individu, on peut par exemple envisager que le premier dispositif de capture d'image soit intégré à une colonne de mesure située dans le magasin de l'opticien et que le deuxième dispositif de capture d'image soit un dispositif d'usage courant, à la disposition de l'individu chez lui, et de préférence pouvant être connecté à internet.

Dans ce cas, le deuxième dispositif de mesure, Kinect ou tablette par exemple, est reliée via un site internet à l'unité de traitement informatique. L'individu peut s'identifier sur ce site par un identifiant et obtenir de l'aide pour la mise en oeuvre de l'étape c) du procédé, par exemple pour le placement du dispositif de capture, les tests à effectuer avant la capture d'images...

Ceci peut être réalisé avant ou après la vente de l'équipement optique.

Les paramètres de comportement visuel sont utilisés pour personnaliser les lentilles ophtalmiques destinées à l'individu. Ils permettent de modifier la lentille ophtalmique standard pour l'adapter au mieux aux besoins de l'individu. Il est également possible de pondérer des paramètres comportementaux déjà déterminés chez l'opticien.

## Revendications

1. Procédé de détermination d'au moins un paramètre de comportement visuel d'un individu, comprenant l'étape suivante :
- détermination de la position du centre de rotation (CRO) d'au moins un oeil (OD) de l'individu dans un premier référentiel lié à la tête de l'individu, **caractérisé en ce que** le procédé comprend en outre les étapes suivantes :
- capture, à l'aide d'un dispositif de capture d'image, d'au moins une image d'au moins une partie du corps de l'individu dans un deuxième référentiel,
- détermination de la position et l'orientation, dans le deuxième référentiel, du premier référentiel lié à la tête (TS) de l'individu, en recherchant la position, dans le deuxième référentiel, d'une zone remarquable de ladite partie du corps de l'individu
- détermination de la position du centre de rotation (CRO) de l'oeil dans le deuxième référentiel,
- détermination du paramètre de comportement visuel recherché en tenant compte de la position du centre de rotation (CRO) de l'oeil déterminée dans le deuxième référentiel.

2. Procédé selon la revendication 1, dans lequel, pour l'étape de capture, on place l'individu dans une situation de vie dans laquelle la posture de la tête (TS) et du corps de l'individu est libre.

3. Procédé selon la revendication 1 ou 2, dans lequel la position et l'orientation de la zone remarquable de ladite partie du corps de l'individu sont prédéterminées dans le premier référentiel.

4. Procédé selon l'une des revendications 1 à 3, selon lequel, à l'étape de détermination de la position du centre de rotation dans le premier référentiel, la position du centre de rotation (CRO) de l'oeil de l'individu est déterminée à partir d'une base de données morphologique de l'individu.

5. Procédé selon l'une des revendications 1 à 3, selon lequel, à l'étape de détermination de la position du centre de rotation dans le premier référentiel, la position du centre de rotation (CRO) de l'oeil de l'individu est déterminée à partir d'une ou plusieurs acquisitions d'images de la tête (TS) de l'individu.

6. Procédé selon l'une des revendications précédentes, selon lequel, à l'étape de capture, ladite image est une représentation en trois dimensions de la partie du corps de l'individu.

7. Procédé selon l'une des revendications 1 à 5, selon lequel à l'étape de capture, ladite image est une représentation en trois dimensions de la tête de l'individu.

8. Procédé selon l'une des revendications 1 à 7, selon lequel, à l'étape de capture, on capture une séquence d'images pendant une durée prédéterminée, et à l'étape de détermination du paramètre de comportement visuel recherché, on en déduit l'évolution du paramètre de comportement visuel recherché pendant cette durée prédéterminée.

9. Procédé selon l'une des revendications 1 à 8, selon lequel, à l'étape de détermination de la position et de l'orientation, ladite zone remarquable est constituée par un système de repérage monté sur la tête (TS) de l'individu, par une monture de lunettes (10) disposée sur sa tête (TS) ou par un ou plusieurs points remarquable du visage de l'individu.

10. Procédé selon l'une des revendications 1 à 9, selon lequel, à l'étape de détermination du paramètre de comportement visuel, on détermine la direction du regard de l'individu dans ladite situation de vie et on en déduit une zone d'usure de la lentille ophtalmique correspondant à cette situation de vie.

11. Procédé selon la revendication 10, selon lequel, pour déterminer la direction du regard de l'individu, on identifie, sur l'image capturée à l'étape de capture, l'image de la pupille de l'individu et on en déduit la direction du regard recherchée, en fonction de la position du centre de rotation (CRO) dans le premier référentiel déterminée à l'étape de détermination de la position du centre de rotation dans le premier référentiel.

12. Procédé selon la revendication 10, selon lequel, pour déterminer la direction du regard de l'individu, on détermine la position dans le deuxième référentiel d'éléments cibles du regard appartenant à l'environnement de l'individu.

13. Procédé selon la revendication 12, selon lequel, lesdits éléments cibles du regard sont affichés sur un écran électronique et ont une position connue par rapport au dispositif de capture d'image.

14. Procédé selon l'une des revendications précédentes, selon lequel, à l'étape de détermination de la position du centre de rotation dans le premier référentiel,
- on réalise, à l'aide d'un dispositif de capture d'image, la capture d'au moins deux images de la tête (TS) de l'individu pour lesquelles les postures de la tête (TS) de l'individu par rapport à ce dispositif de capture d'image sont différentes et pour lesquelles l'individu fixe du regard un point de visée de position prédéterminée,
- on détermine les directions de regard de l'individu correspondant à chacune des deux images,
- on en déduit la position du centre de rotation (CRO) de l'oeil de l'individu.

15. Procédé selon l'une des revendications précédentes, selon lequel, à l'étape de détermination de la position du centre de rotation dans le premier référentiel, la tête (TS) de l'individu est munie d'une paire de lunettes surmontée d'un système de repérage, à l'étape de capture, la partie du corps capturée comprend la tête (TS) de l'individu munie de ce système de repérage, et, à l'étape de détermination de la position et de l'orientation, la zone remarquable est constituée par ce système de repérage.

16. Procédé selon l'une des revendications précédentes, selon lequel, le paramètre de comportement visuel de l'individu recherché est l'un des suivants :
- une zone d'usure correspondant à la situation de vie de l'étape b) pour une lentille correctrice à disposer dans une monture de lunettes (10) destinée à équiper la tête de l'individu,
- un paramètre comportemental de l'individu précisant s'il bouge plus les yeux ou la tête (TS),
- distance de lecture moyenne,
- posture naturelle de l'individu au repos,
- comportement dynamique des yeux pendant la situation de vie choisie,
- position d'une zone de vision de près ou longueur de progression ou inset d'une lentille correctrice à disposer dans une monture de lunettes (10) destinée à équiper la tête (TS) de l'individu,
- l'angle pantoscopique d'une lentille correctrice à disposer dans une monture de lunettes (10) destinée à équiper la tête (TS) de l'individu déterminé de manière à réduire les aberration d'astigmatisme.

17. Procédé selon l'une des revendications précédentes, selon lequel les étapes de détermination de la position du centre de rotation dans le premier référentiel et de capture sont réalisées en un même lieu, à l'aide d'un même dispositif de capture d'image.

18. Procédé selon l'une des revendications précédentes, selon lequel les étapes de détermination de la position du centre de rotation dans le premier référentiel et de capture sont réalisées en deux lieux différents, à l'aide de deux dispositifs de capture d'image différents.

## Patentansprüche

1. Verfahren zur Bestimmung mindestens eines Sehverhalten-Parameters einer Person, das den folgenden Schritt enthält:
- Bestimmung der Position des Drehpunkts (CRO) mindestens eines Auges (OD) der Person in einem mit dem Kopf der Person verbundenen ersten Bezugssystem,
**dadurch gekennzeichnet, dass** das Verfahren außerdem die folgenden Schritte enthält:
- Aufnahme, mit Hilfe einer Bildaufnahmevorrichtung, mindestens eines Bilds mindestens eines Teils des Körpers der Person in einem zweiten Bezugssystem,
- Bestimmung der Position und der Ausrichtung des mit dem Kopf (TS) der Person verbundenen ersten Bezugssystems im zweiten Bezugssystem, indem die Position einer Kennzone des Teils des Körpers der Person im zweiten Bezugssystem gesucht wird,
- Bestimmung der Position des Drehpunkts (CRO) des Auges im zweiten Bezugssystem,
- Bestimmung des gesuchten Sehverhalten-Parameters unter Berücksichtigung der im zweiten Bezugssystem bestimmten Position des Drehpunkts (CRO) des Auges.

2. Verfahren nach Anspruch 1, wobei für den Aufnahmeschritt die Person in einer Lebenssituation platziert wird, in der die Haltung des Kopfes (TS) und des Körpers der Person frei ist.

3. Verfahren nach Anspruch 1 oder 2, wobei die Position und die Ausrichtung der Kennzone des Teils des Körpers der Person im ersten Bezugssystem vorbestimmt sind.

4. Verfahren nach einem der Ansprüche 1 bis 3, wobei im Bestimmungsschritt der Position des Drehpunkts im ersten Bezugssystem die Position des Drehpunkts (CRO) des Auges der Person ausgehend von einer morphologischen Datenbank der Person bestimmt wird.

5. Verfahren nach einem der Ansprüche 1 bis 3, wobei im Bestimmungsschritt der Position des Drehpunkts im ersten Bezugssystem die Position des Drehpunkts (CRO) des Auges der Person ausgehend von einer oder mehreren Bilderfassungen des Kopfs (TS) der Person bestimmt wird.

6. Verfahren nach einem der vorhergehenden Ansprüche, wobei im Aufnahmeschritt das Bild eine dreidimensionale Darstellung des Teils des Körpers der Person ist.

7. Verfahren nach einem der Ansprüche 1 bis 5, wobei im Aufnahmeschritt das Bild eine dreidimensionale Darstellung des Kopfs der Person ist.

8. Verfahren nach einem der Ansprüche 1 bis 7, wobei im Aufnahmeschritt eine Bildfolge während einer vorbestimmten Dauer aufgenommen wird, und im Bestimmungsschritt des gesuchten Sehverhalten-Parameters daraus die Entwicklung des gesuchten Sehverhalten-Parameters während dieser vorbestimmten Dauer abgeleitet wird.

9. Verfahren nach einem der Ansprüche 1 bis 8, wobei im Bestimmungsschritt der Position und der Ausrichtung die Kennzone aus einem auf den Kopf (TS) der Person montierten Markierungssystem, aus einem auf seinem Kopf (TS) angeordneten Brillengestell (10) oder aus einem oder mehreren Kennpunkten des Gesichts der Person besteht.

10. Verfahren nach einem der Ansprüche 1 bis 9, wobei im Bestimmungsschritt des Sehverhalten-Parameters die Richtung des Blicks der Person in der Lebenssituation bestimmt und daraus eine Abnutzungszone der ophthalmischen Linse entsprechend dieser Lebenssituation abgeleitet wird.

11. Verfahren nach Anspruch 10, wobei zur Bestimmung der Richtung des Blicks der Person auf dem im Aufnahmeschritt aufgenommenen Bild das Bild der Pupille der Person erkannt und daraus die gesuchte Richtung des Blicks abhängig von der Position des Drehpunkts (CRO) im ersten Bezugssystem, die im Bestimmungsschritt der Position des Drehpunkts im ersten Bezugssystem bestimmt wird, abgeleitet wird.

12. Verfahren nach Anspruch 10, wobei zur Bestimmung des Blicks der Person die Position im zweiten Bezugssystem von Zielelementen des Blicks bestimmt wird, die zur Umgebung der Person gehören.

13. Verfahren nach Anspruch 12, wobei die Zielelemente des Blicks auf einem elektronischen Bildschirm angezeigt werden und eine bekannte Position bezüglich der Bildaufnahmevorrichtung haben.

14. Verfahren nach einem der vorhergehenden Ansprüche, wobei im Bestimmungsschritt der Position des Drehpunkts im ersten Bezugssystem
- mit Hilfe einer Bildaufnahmevorrichtung die Aufnahme von mindestens zwei Bildern des Kopfs (TS) der Person durchgeführt wird, für die die Haltungen des Kopfs (TS) der Person bezüglich dieser Bildaufnahmevorrichtung unterschiedlich sind und für die die Person einen Zielpunkt vorbestimmter Position mit dem Blick fixiert,
- die Blickrichtungen der Person entsprechend jedem der zwei Bilder bestimmt werden,
- daraus die Position des Drehpunkts (CRO) des Auges der Person abgeleitet wird.

15. Verfahren nach einem der vorhergehenden Ansprüche, wobei im Bestimmungsschritt der Position des Drehpunkts im ersten Bezugssystem der Kopf (TS) der Person mit einer Brille versehen wird, auf die ein Markierungssystem aufgesetzt ist, im Aufnahmeschritt der aufgenommene Teil des Körpers den mit diesem Markierungssystem versehenen Kopf (TS) der Person enthält, und im Bestimmungsschritt der Position und der Ausrichtung die Kennzone aus diesem Markierungssystem besteht.

16. Verfahren nach einem der vorhergehenden Ansprüche, wobei der gesuchte Sehverhalten-Parameter der Person einer der folgenden ist:
- eine Abnutzungszone entsprechend der Lebenssituation des Schritts b) für eine Korrekturlinse, die in einem Brillengestell (10) anzuordnen ist, das dazu bestimmt ist, den Kopf der Person auszustatten,
- ein Verhaltensparameter der Person, der präzisiert, ob sie mehr die Augen oder den Kopf (TS) bewegt,
- der mittlere Leseabstand,
- die natürliche Haltung der Person in der Ruhestellung,
- das dynamische Verhalten der Augen während der gewählten Lebenssituation,
- die Position einer Nahsichtzone oder Progressionslänge oder Inset einer Korrekturlinse, die in einem Brillengestell (10) anzuordnen ist, das dazu bestimmt ist, den Kopf (TS) der Person auszustatten,
- der pantoskopische Winkel einer Korrekturlinse, die in einem Brillengestell (10) anzuordnen ist, das dazu bestimmt ist, den Kopf (TS) der Person auszustatten, festgelegt, um die Astigmatismus-Aberrationen zu verringern.

17. Verfahren nach einem der vorhergehenden Ansprüche, wobei die Schritte der Bestimmung der Position des Drehpunkts im ersten Bezugssystem und der Aufnahme am gleichen Ort mit Hilfe einer gleichen Bildaufnahmevorrichtung durchgeführt werden.

18. Verfahren nach einem der vorhergehenden Ansprüche, wobei die Schritte der Bestimmung der Position des Drehpunkts im ersten Bezugssystem und der Aufnahme an zwei verschiedenen Orten mit Hilfe von zwei unterschiedlichen Bildaufnahmevorrichtungen durchgeführt werden.

## Claims

1. Method for determining at least one parameter of visual behavior of an individual, comprising the following step:
- determining the position of the rotation center (CRO) of at least one eye (OD) of the individual in a first frame of reference associated with the head of the individual,
**characterised in that** the method further comprises the following steps:
- capturing, using an image-capturing device, at least one image of at least one portion of the body of the individual in a second frame of reference,
- determining the position and orientation, in the second frame of reference, of the first frame of reference associated with the head (TS) of the individual, by seeking the position, in the second frame of reference, of a recognizable zone of said portion of the body of the individual,
- determining the position of the rotation center (CRO) of the eye in the second frame of reference,
- determining the sought parameter of visual behavior while taking into account the position of the rotation center (CRO) of the eye, which position is determined in the second frame of reference.

2. Method according to Claim 1, wherein, for the capturing step, the individual is placed in a real-life situation in which the posture of the head (TS) and body of the individual is unconstrained.

3. Method according to Claim 1 or 2, wherein the position and orientation of the recognizable zone of said portion of the body of the individual are predetermined in the first frame of reference.

4. Method according to one of Claims 1 to 3, wherein, in the step of determining the position of the rotation center in the first frame of reference, the position of the rotation center (CRO) of the eye of the individual is determined from a morphological database of the individual.

5. Method according to one of Claims 1 to 3, wherein, in the step of determining the position of the rotation center in the first frame of reference, the position of the rotation center (CRO) of the eye of the individual is determined from one or more acquisitions of images of the head (TS) of the individual.

6. Method according to one of the preceding claims, wherein, in the capturing step, said image is a three-dimensional representation of the portion of the body of the individual.

7. Method according to one of Claims 1 to 5, wherein, in the capturing step, said image is a three-dimensional representation of the head of the individual.

8. Method according to one of Claims 1 to 7, wherein, in the capturing step, a sequence of images is captured during a predetermined duration, and, in the step of determining the sought parameter of visual behavior, the variation in the sought parameter of visual behavior during this predetermined duration is deduced therefrom.

9. Method according to one of Claims 1 to 8, wherein, in the step of determining position and orientation, said recognizable zone consists of a pinpointing system mounted on the head (TS) of the individual, of a spectacle frame (10) placed on the head (TS) or of one or more recognizable points of the face of the individual.

10. Method according to one of Claims 1 to 9, wherein, in the step of determining the parameter of visual behavior, the direction of the gaze of the individual in said real-life situation is determined, and a zone of use of the ophthalmic lens corresponding to this real-life situation is determined therefrom.

11. Method according to Claim 10, wherein, to determine the direction of the gaze of the individual, the image of the pupil of the individual is identified in the image captured in the capturing step, and the sought direction of the gaze is deduced therefrom depending on the position of the rotation center (CRO) in the first frame of reference, said position being determined in the step of determining the position of the rotation center in the first frame of reference.

12. Method according to Claim 10, wherein, to determine the direction of the gaze of the individual, the position in the second frame of reference of elements targeted by the gaze and belonging to the environment of the individual is determined.

13. Method according to Claim 12, wherein, said elements targeted by the gaze are displayed on an electronic screen and have a known position with respect to the image-capturing device.

14. Method according to one of the preceding claims, wherein, in the step of determining the position of the rotation center in the first frame of reference,
- at least two images of the head (TS) of the individual are captured using an image-capturing device, in which images the postures of the head (TS) of the individual with respect to this image-capturing device are different and in which images the individual is fixating his gaze on a sighting point of predetermined position,
- the gaze directions of the individual corresponding to each of the two images are determined,
- the position of the rotation center (CRO) of the eye of the individual is deduced therefrom.

15. Method according to one of the preceding claims, wherein, in the step of determining the position of the rotation center in the first frame of reference, the head (TS) of the individual is equipped with a pair of spectacles surmounted by a pinpointing system, in the capturing step, the captured portion of the body comprises the head (TS) of the individual equipped with this pinpointing system, and, in the step of determining the position and orientation, the recognizable zone consists of this pinpointing system.

16. Method according to one of the preceding claims, wherein the sought parameter of visual behavior of the individual is one of the following:
- a zone of use corresponding to the real-life situation of step b) for a corrective lens to be placed in a spectacle frame (10) intended to equip the head of the individual,
- a behavioral parameter of the individual specifying whether he moves his eyes or head (TS) more,
- average reading distance,
- natural posture of the individual at rest,
- dynamic behavior of the eyes during the chosen real-life situation,
- position of a near-vision zone or progression length or inset of a corrective lens to be placed in a spectacle frame (10) intended to equip the head (TS) of the individual,
- the pantoscopic angle of a corrective lens to be placed in a spectacle frame (10) intended to equip the head (TS) of the individual determined so as to decrease the astigmatic aberrations.

17. Method according to one of the preceding claims, wherein the steps of determining the position of the rotation center in the first frame of reference and of capturing are carried out in one and the same place, using one and the same image-capturing device.

18. Method according to one of the preceding claims, wherein the steps of determining the position of the rotation center in the first frame of reference and of capturing are carried out in two different places, using two different image-capturing devices.
